# EUROPÄISCHE PATENTSCHRIFT

(11) **EP 0 563 783 B1**
(45) Veröffentlichungstag und Bekanntmachung des Hinweises auf die Patenterteilung: **13.11.1996**
(21) Anmeldenummer: 93104902.7
(22) Anmeldetag: 24.03.1993
(51) Int. Cl.: A61F 2/16, G02C 7/04

(54) **Intraokularlinsenset**
Intraocular lens set
Jeu de lentilles intra-oculaires

(30) Priorität: 03.04.1992 EP 92105814
(43) Veröffentlichungstag der Anmeldung: 06.10.1993
(73) Patentinhaber: Chiron Adatomed Pharmazeutische und Medizintechnische Gesellschaft mbH, D-85609 Dornach (DE)
(72) Erfinder: Jacobi, Karl Wilhelm, Prof., W-6300 Giessen (DE)
(74) Vertreter: Nöth, Heinz, Dipl.-Phys.

(56) Entgegenhaltungen:
- EP-A- 0 140 063
- EP-A- 0 453 136
- WO-A-89/02251
- WO-A-90/00889
- WO-A-91/09336
- US-A- 5 089 024

## Beschreibung

Die Erfindung betrifft ein Intraokularlinsenset, bestehend aus zwei multifokalen Intraokularlinsen.

Beim Einsatz von multifokalen Intraokularlinsen, z.B. zur Behandlung von grauem Star, nimmt man eine geringere Kontrastempfindlichkeit in Kauf. Insbesondere bei Nachtfahrten mit dem Auto erwachsen hieraus Schwierigkeiten. Beim Einsatz von Intraokularlinsen mit einer zentralen Linsenzone für das Distanzsehen ergibt sich weiterhin eine hohe Abhängigkeit der durch den jeweiligen Linsenbereich einfallenden Lichtmenge von der Pupillengröße.

Aus der EP-A-453 136 ist ein Kontaktlinsenpaar aus zwei bifokalen Linsen bekannt, wobei jede Linse für den Nah- und Fernbereich eine unterschiedliche Lichtverteilung hat. Die Lichtverteilung ist dabei so, daß in demjenigen Fokussierungsbereich (fern oder nah), in dem die eine Linse eine hohe Lichtintensität aufweist, die andere Linse eine niedrige Lichtintensität hat und umgekehrt. Die Linsen besitzen hierzu refraktive und diffraktive Anteile.

Aus der WO-A-91-9336 ist ein Kontaktlinsenpaar aus zwei multifokalen, insbesondere bifokalen Linsen bekannt, bei denen die höhere der beiden Lichtintensitäten 50 - 100 % der Gesamtlichtverteilung ausmacht.

Aufgabe der Erfindung ist es, ein Intraokularlinsenset der eingangs genannten Art zu schaffen, bei dem die Kontrastempfindlichkeit sowie das Sehvermögen bei reduzierten Lichtverhältnissen (Dämmerung, Nacht) verbessert ist.

Diese Aufgabe wird erfindungsgemäß durch die Merkmale des Patentanspruches 1 gelöst.

In bevorzugter Weise kann der jeweils höhere Anteil der Lichtverteilung für den Fern- bzw. Nahfokus bei den beiden Intraokularlinsen 60% - 70% der Gesamtlichtverteilung, welche durch die jeweilige Linse hindurchtritt, betragen. Die beiden Linsen können als im wesentlichen bifokale Linsen ausgebildet sein. Dabei kann es sich um solche Linsen handeln, die an ihrer einen Oberflächenseite als brechende Linse und an ihrer anderen Oberflächenseite als Streulinse ausgebildet sind. In bekannter Weise läßt sich durch die Streuwirkung d.h. durch Interferenz von in Phase befindlichen Lichtwellen die Lichtintensität am jeweiligen Brennpunkt (Fernfokus oder Nahfokus) der Linse erhöhen. In diesem Zusammenhang wird beispielsweise auf die deutsche Patentanmeldung DE-A-4134518 hingewiesen. Die beiden Linsen können jedoch auch in der Weise ausgebildet sein wie es aus der EP 0140063B1 bekannt ist, wobei am Linsenkörper zur Erzielung des höheren Anteils an Lichtverteilung entsprechend mehr Linsenzonen für den Nahbereich bzw. Fernbereich vorgesehen sind.

Anhand der Figuren wird die Erfindung noch näher erläutert. Es zeigt:
- Fig. 1: in schematischer Darstellung zwei bifokale Intraokularlinsen mit den zugehörigen Fern- bzw. Nahbrennweiten mit asymetrischer Lichtverteilung auf den Fern- und Nahbereich der jeweiligen Linse;
- Fig. 2: eine Darstellung der jeweiligen Linse im Nah- bzw. Fernbereich;
- Fig. 3: das kombinatorische Zusammenwirken der beiden Linsen;
- Fig. 4: zeigt ein Kontrastempfindlichkeitsdiagramm in verschiedenen Abständen nach Regan für zwei bifokale Intraokularlinsen, welche Bestandteil eines Ausführungsbeispieles der Erfindung sind und
- Fig. 5: ein Konstrastempfindlichkeitsdiagramm in verschiedenen Distanzen nach Pelli-Robson für zwei bifokale Intraokularlinsen, die Bestandteil eines Ausführungsbeispieles sind.

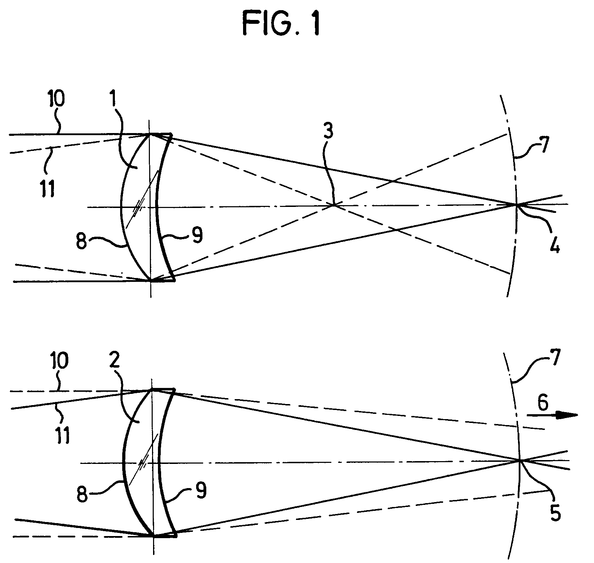

In der Fig.1 sind zwei bifokale Intraokularlinsen 1 und 2 dargestellt, welche ein Intraokularlinsenset nach einem Ausführungsbeispiel der Erfindung bilden. Die Intraokularlinse 1 besitzt einen Fernfokus 4, in welchem parallele aus der Ferne kommende Lichtstrahlen 10 fokussiert werden. Aus der Nähe einfallende Lichtstrahlen 11 werden in einem Nahfokus 3 gesammelt.

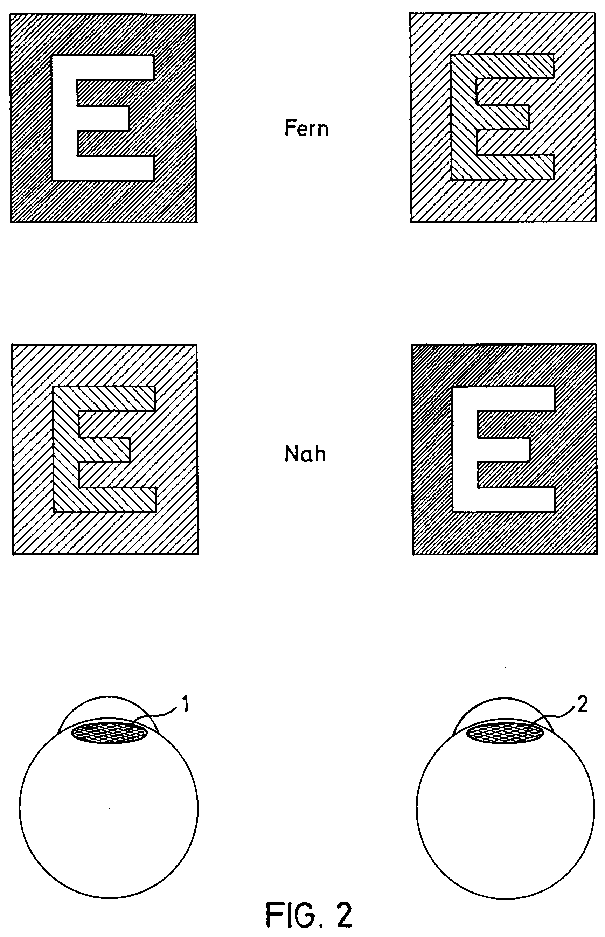

Die einfallende Lichtenergie wird mit einem größeren Anteil (60% - 70%) im Fernfokus 4 fokussiert als im Nahfokus 3. Der Fernfokus 4 liegt bei der implantierten Linse auf der Retina des Auges, welche in der Fig.1 durch die strichpunktierte Linie 7 kenntlich gemacht ist. Der Anteil an Lichverteilung im Nahfokus 3 beträgt 30% - 40%. Das im Fernfokus 4 gesammelte Licht erzeugt ein scharfes Bild auf der Retina des Auges. Die Lichtintensität des fokussierten aus der Ferne kommenden Lichtes übersteigt erheblich die Lichtintensität des aus der Nähe kommenden auf der Retina defokussierten Lichtes. Dieses aus der Nähe kommende Licht erzeugt auf der Retina ein nur äußerst schwaches unstrukturiertes Bild. In der Fig.2 ist das im Fernfokus 4 und das durch den Nahfokus 3 abgebildete Licht für die Intraokularlinse 1 (linke in ein Auge implantierte Linse) dargestellt. Für das aus der Ferne kommende Bild (E) ergibt sich eine Abbildung mit guter Schärfe und mit gutem Kontrast während für das Nahbild entsprechend dem geringeren Anteil an der Lichtverteilung eine geringere Intensität sich ergibt.

Das Intraokularlinsenset besitzt eine zweite Intraokularlinse 2, deren Nahfokus 5 im implantierten Zustand auf der Retina des Auges (strichpunktierte Linie 7) liegt. Bei der Intraokularlinse 2 ist ein größerer Anteil (60% - 70%) der Lichtenergie, welche durch die Intraokularlinse 2 hindurchtritt fokussiert als im hinter der Retina liegenden Fernfokus 6.Im Nahfokus 5 wird das aus der Nähe in die Intraokularlinse 2 einfallende Licht 11 gesammelt. Im hinter der Retina 7 liegenden Fernfokus 6 wird das parallel in die Intraokularlinse 2 einfallende Licht 10 aus der Ferne gesammelt. Durch den Nahfokus 5 der Linse 2 (rechte im Auge implantierte Linse) wird, wie die Fig.2 zeigt, vom Nahbereich ein scharfes Bild dargestellt, während vom Fernbereich ein relativ intensitätsschwaches Bild, das dem geringeren Anteil an Lichtvertilung entspricht, gebildet wird.

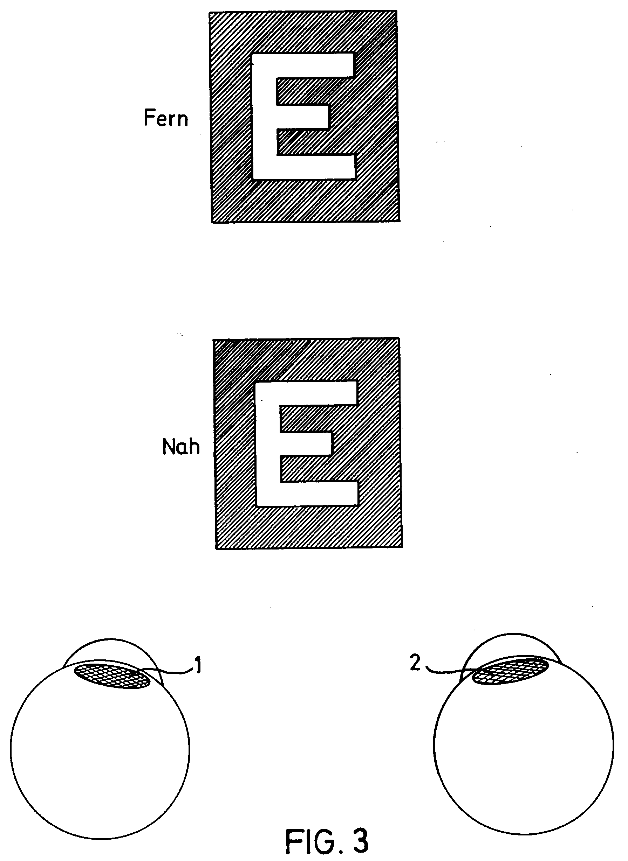

Aus der Fig. 3 ist zu ersehen, daß durch das kombinatorische Zusammenwirken der beiden implantierten Linsen 1 und 2 sowohl für die Ferne als auch für die Nähe scharfe, kontrastreiche Bilder mit hoher Intensität sich erreichen lassen.

Zur Überprüfung der Sehfähigkeit bei Einsatz eines Intraokularlinsensets gemäß einem Ausführungsbeispiel der Erfindung wurden die Defokussierungskurven zweier Intraokularlinsen untersucht. Die eine Intraokularlinse (Linse für das rechte Auge) besitzt für den Nahfokus einen Anteil der Lichtverteilung von 60% und für den Fernfokus einen Anteil der Lichtverteilung von 40%. Die andere Intraokularlinse (Linse für das linke Auge) besitzt für den Nahfokus einen Anteil für die Lichtverteilung von 40% und für den Fernfokus einen Anteil an der Lichtverteilung von 60%.

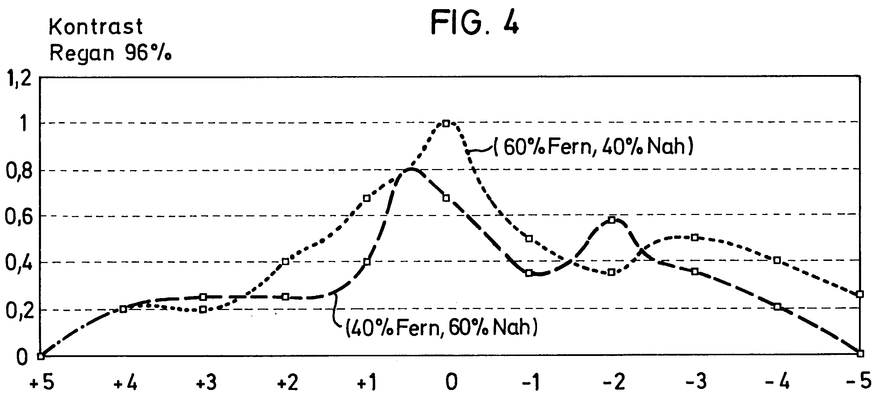

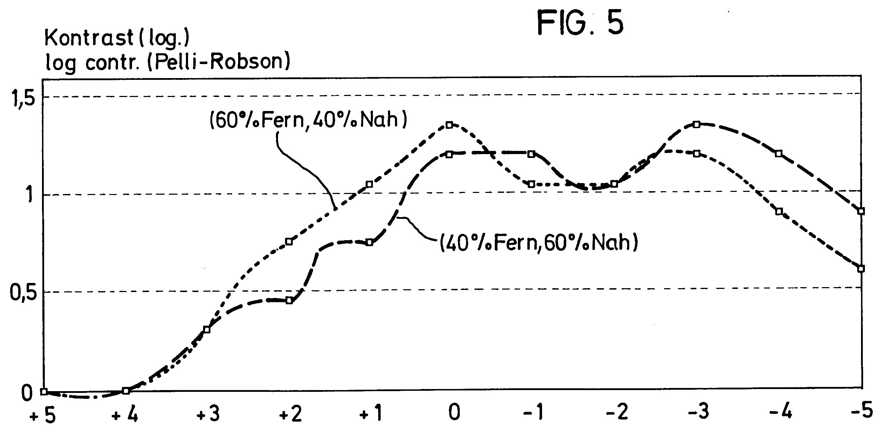

In der Fig.4 sind für die beiden Linsen die Defokussierungskurven für die Sehschärfe nach Regan 96 % dargestellt. In der Fig. 5 sind die Defokussierungskurven für die beiden Linsen bezüglich des Sehkontrastes nach Pelli-Robson dargestellt.

Die beiden in einem Intraokularlinsenset zum Einsatz kommenden Linsen können so ausgebildet sein, daß ihre Brechkraft aus einem diffraktiven und einem refraktiven Anteil zusammengesetzt ist. Derartige Intraokularlinsen sind in der deutschen Patentanmeldung P4134518.5 beschrieben. Bei derartig ausgebildeten Linsen kann der Brechkraft des refraktiven Linsenteils noch die zusätzliche diffraktive Brechkraft hinzugefügt werden, so daß einer der beiden Foki im entsprechenden Umfang unterdrückt wird. Für die diffraktive Feinstruktur an der Rückseite 9 der Linse 1 bzw. 2 kann ein Sägezahnprofil zum Einsatz kommen, wobei die Profilhöhe bzw. die Differenz der optischen Wellenlänge zwischen Spitze und Tal der Designwellenlänge bevorzugt einer Wellenlänge im grünen Spektralbereich des sichtbaren Lichtes entspricht. In diesem Fall kann die Wirkung des diffraktiven Feinstrukturprofils an der Rückseite 9 der Linse 1 bzw. 2 so eingestellt werden, daß sich die Linse der Wirkung einer monofokalen Linse annähert. Der refraktive Anteil der Linse 1 bzw. 2 wird jeweils an ihrer Vorderseite 8 gebildet.

## Patentansprüche

1. Intraokularlinsenset mit einer ersten Intraokularlinse (1) und einer zweiten Intraokularlinse (2), von denen jede Linse einen Nahfokus (3, 5) und einen Fernfokus (4, 6) aufweist und bei denen die jeweilige Brechkraft aus einem diffraktiven und refraktiven Anteil zusammengesetzt ist, wobei der diffraktive Anteil an der Rückseite (9) jeder Linse von einem diffraktiven Strukturprofil gebildet ist, dessen Profilhöhe einer Wellenlänge im grünen Spektralbereich des sichtbaren Lichtes entspricht und wobei die diffraktiven und refraktiven Anteile in der Weise addiert sind, daß die erste Linse (1) für ihren Fernfokus (4) einen höheren Anteil an Lichtverteilung hat als die zweite Linse (2) und die zweite Linse (2) für ihren Nahfokus (5) einen höheren Anteil an der Lichtverteilung hat als die erste Linse (1).

2. Intraokularlinsenset nach Anspruch 1, dadurch gekennzeichnet, daß der jeweils höhere Anteil der Lichtverteilung für den Fern- bzw. Nahfokus (4 bzw. 5) bei den beiden Intraokularlinsen (1, 2) 60% bis 70% der Gesamtlichtverteilung, die durch die jeweilige Linse hindurchtritt, beträgt.

## Claims

1. An intraocular lens set having a first intraocular lens (1) and a second intraocular lens (2) of which each lens has a near focus (3, 5) and a far focus (4, 6) and in which the respective refractive power is composed of a diffractive and refractive component, wherein the diffractive component is formed at the rear side (9) of each lens by a diffractive structure profile whose profile height corresponds to a wavelength in the green spectral region of visible light, and wherein the diffractive and refractive components are added in such a way that the first lens (1) has for its far focus (4) a higher proportion of light distribution than the second lens (2) and the second lens (2) has for its near focus (5) a higher proportion of light distribution than the first lens (1).

2. An intraocular lens set according to claim 1 characterised in that the respective higher proportion of light distribution for the far and near focus (4 and 5) respectively in the case of the two intraocular lenses (1, 2) is 60% to 70% of the total light distribution which passes through the respective lens.

## Revendications

1. Jeu de lentilles intra-oculaires comportant une première lentille intra-oculaire (1) et une seconde lentille intraoculaire (2), dont chacune présente un punctum proximum (3, 5) et un punctum remotum (4, 6), et sur lesquelles le pouvoir réfringent respectif se compose d'une part de diffraction et d'une part de réfraction, la part de diffraction étant formée sur la face arrière (9) de chaque lentille par un profil structuré de diffraction, dont la hauteur de profil correspond à une longueur d'onde dans le domaine spectral vert de la lumière visible, et les parts de diffraction et de réfraction étant additionnées de sorte que la première lentille (1) présente pour son punctum remotum (4) une part de répartition de lumière plus élevée que la seconde lentille (2), la seconde lentille (2) présentant pour son punctum proximum (5) une part de répartition lumineuse plus élevée que la première lentille (1).

2. Jeu de lentilles intra-oculaires suivant la revendication 1, caractérisé en ce que la part respectivement supérieure de la répartition de lumière pour le punctum remotum et/ou proximum (4 et/ou 5) atteint sur les deux lentilles intra-oculaires (1, 2) 60 % à 70 % de la répartition totale de lumière, passant au travers de la lentille respective.
